(19) Europäisches Patentamt
European Patent Office
Office européen des brevets

(11) **EP 3 824 927 A1**

(12) **EUROPEAN PATENT APPLICATION**

(43) Date of publication:
**26.05.2021 Bulletin 2021/21**

(51) Int Cl.:
**A61M 5/31** (2006.01)

(21) Application number: **19210764.7**

(22) Date of filing: **21.11.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: **Schott AG**
**55122 Mainz (DE)**

(72) Inventors:
• **Moser, Raymond**
  **9032 Engelburg (CH)**
• **Mangold, Stephanie**
  **55288 Schornsheim (DE)**

(74) Representative: **Herzog IP Patentanwalts GmbH**
**Steinstraße 16-18**
**40212 Düsseldorf (DE)**

(54) **RECEPTACLE FOR PHARMACEUTICAL PACKAGING WITH A THICKNESS PROFILE FOR A LUBRICANT LAYER**

(57) The invention generally relates to a receptacle (100) for pharmaceutical packaging characterised by a thickness profile for a lubricant layer (106). In particular, the invention relates to a receptacle; a kit comprising a receptacle and a charge (203); an assembly comprising a receptacle, a charge and a liquid pharmaceutical composition; a process for the preparation of a receptacle and a use of a lubricant layer characterised by its thickness profile.

Figure 1

EP 3 824 927 A1

**Description**

Field of the Invention

The invention generally relates to a receptacle for pharmaceutical packaging characterised by a thickness profile for a lubricant layer. In particular, the invention relates to a receptacle; a kit comprising a receptacle and a charge; an assembly comprising a receptacle, a charge and a liquid pharmaceutical composition; a process for the preparation of a receptacle and a use of a lubricant layer characterised by its thickness profile.

Background

Pharmaceutical material can be provided in a number of forms and contained in a variety of different containers. In the case of a liquid pharmaceutical material, some common examples are ampules, vials, cartridges and syringes. One widely used format employs a sliding plunger within a container for ejecting a liquid out of an aperture. One approach is to provide a lubricating layer on the inside of the container to facilitate sliding movement of the plunger.
U.S. Patent Application Document No. 4,767,414 A describes plasma activation of an inner surface prior to application of a layer of silicone lubricant.
European Patent No. EP 0920879 B1 describes a recipe for a silicone-based mixture comprising reactive components and unreactive components.
There persists a need for improved approaches to lubrication of pharmaceutical containers, in particular for single-dose delivery.

Summary of the Invention

It is an object of the invention to at least partially solve one or more of the above-mentioned challenges.
It is an object of the invention to provide an improved receptacle for pharmaceutical packaging, in particular for single-dose delivery.
It is an object of the invention to provide an improved kit comprising a receptacle and a charge, in particular for single-dose delivery.
It is an object of the invention to provide an improved process for preparing a receptacle for pharmaceutical packaging, in particular for single-dose delivery.
It is an object of the invention to provide an improved assembly comprising a receptacle, a charge and a liquid pharmaceutical composition, in particular for single-dose delivery.
It is an object of the invention to provide a receptacle for pharmaceutical packaging having improved dynamic friction properties, in particular for single-dose delivery.
It is an object of the invention to provide a kit having improved dynamic friction properties, in particular for single-dose delivery.
It is an object of the invention to provide a process for preparing a receptacle for pharmaceutical packaging providing improved dynamic friction properties, in particular for single-dose delivery.
It is an object of the invention to provide an assembly having improved dynamic friction properties, in particular for single-dose delivery.

Detailed Description

The following embodiments, with the $x^{th}$ embodiment labelled as |x|, represent preferred arrangements for approaching the objects of the invention.

|1| A receptacle for pharmaceutical packaging, the receptacle having an elongate barrel section, wherein:

a. the elongate barrel section has a direction of elongate extension and an axis in the direction of elongate extension;
b. an axial position p is determined along the axis;
c. the elongate barrel section extends from an axial position $p_A$ to an axial position $p_B$;
d. an elongate barrel section length $L_B$ is the distance between $p_A$ and $p_B$;
e. the receptacle has a side wall extending over the elongate barrel section, the side wall having an inner surface bordering an interior, the interior having a diameter;
f. a layer of a lubricant is located on at least a part of the inner surface;
g. at a given axial position p on the axis between $p_A$ and $p_B$, the following:

i. the thickness of the side wall,
ii. the thickness of the layer, and
iii. the diameter of the interior,

are each determined as an angular mean in a cross-sectional plane perpendicular to the axis at the axial position p;

h. a portion X of the axis extends from an axial position $p_1$ to an axial position $p_2$ such that the following criteria are satisfied:

i. both $p_1$ and $p_2$ lie between $p_A$ and $p_B$,
ii. a portion length Lx is the distance between pi and $p_2$,
iii. Lx is at least a quarter of $L_B$, , preferably at least a half of $L_B$, more preferably at least 80% of $L_B$, most preferably at least 90% of $L_B$, and
iv. the layer extends over the entire portion X,

i. $T_{mean}$ is a mean thickness of the layer determined in the portion X;
j. a position $p_m$ is the midpoint between $p_1$ and $p_2$;
k. $T_A$ is a mean thickness of the layer for a section from pi to $p_m$;
l. $T_B$ is a mean thickness of the layer for a section from $p_m$ to $p_2$;
m. The ratio $T_A:T_B$ is in the range from 5:1 to 1:5, preferably in the range from 3:1 to 1:3, more preferably in the range from 2:1 to 1:2, most preferably in the range from 4:3 to 3:4;
n. one or more receptacle criteria selected from the group consisting of:

i. The length $L_B$ is in the range from 3 to 20 cm, preferably in the range from 4 to 15 cm, more preferably in the range from 5 to 12 cm, most preferably 6 to 8 cm;
ii. The mean value of the diameter of the interior determined over the range $p_A$ to $p_B$ is in the range from 0.4 to 4 cm, preferably in the range from 0.6 to 3 cm, more preferably in the range from 0.8 to 2.5 cm, most preferably 1 to 2 cm;
iii. The mean thickness of the sidewall determined over the range $p_A$ to $p_B$ is in the range from 0.3 to 4 mm, preferably in the range from 0.7 to 3 mm, more preferably in the range from 1.1 to 2 mm, most preferably 1.4 to 1.8 mm; and
iv. The volume of the interior is in the range from 0.1 to 150 ml, preferably in the range from 0.5 to 50 ml, more preferably in the range from 1 to 25 ml, most preferably 2 to 10 ml, most preferably 3 to 5 ml;

are satisfied.

In one aspect of this embodiment, $T_{mean}$ is in the range from 50 nm to 4100 nm.
In various aspects of this embodiment, the following combinations of the receptacle criteria i. to iv. are fulfilled:
i., ii., iii., iv., i.+ii., i.+iii., i.+iv., ii.+iii., ii.+iv., iii.+iv., i.+ii.+iii., i.+ii.+iv., i.+iii.+iv., ii.+iii.+iv & i.+ii.+iii.+iv..

|2| The receptacle according to embodiment |1|, wherein the mean thickness $T_{mean}$ of the layer is in the range from 100 nm to 3000 nm, preferably in the range from 300 to 2000 nm, more preferably in the range from 500 to 1500 nm, most preferably in the range from 700 to 1200 nm.

|3| The receptacle according to embodiment |1| or |2|, wherein:

a. the inner surface of the side wall has a roughness $r_{rms}$, and
b. the ratio $r_{rms} : T_{mean}$ is in the range from 1:2 to 1:20, preferably in the range from 1:3 to 1:15, more preferably in the range from 1:4 to 1:10.

|4| The receptacle according to any of the preceding claims, wherein the layer has a maximum layer thickness $T_{max}$ determined between $p_1$ and $p_2$, a minimum layer thickness Tmin determined between $p_1$ and $p_2$ and the value of $T_{min} / T_{max}$ is at least 0.7, preferably ate least, 0.75, more preferably 0.8, most preferably 0.9, most preferably 0.95.

|5| The receptacle according to any of the preceding claims, wherein the side wall comprises a plastic or a glass or both.

|6| The receptacle according to any of the preceding embodiments, wherein the lubricant comprises one or more silicone oils. Preferably, the lubricant comprises in total at least 5 wt. %, more preferably at least 15 wt. %, most preferably at least 25 wt. % of one or more silicone oils, based on the total weight of the lubricant of the layer.

|7| The receptacle according to embodiment |6|, wherein the one or more silicone oils are at least partially contained in a matrix, wherein the matrix is bound to the inner surface.

|8| The receptacle according to embodiment |7|, wherein the matrix is a polymer, preferably a crosslinked polymer.

|9| The receptacle according to embodiment |8|, wherein the polymer comprises SiO-containing repeating units. A preferred polymer is a polysiloxane, more preferably a crosslinked polysiloxane.

|10| The receptacle according to any of the preceding embodiments, wherein the interior is cylindrical or truncated conical over the elongate barrel section. A preferred truncated cone has a cone aperture in the range from 0.04° to 0.4°, preferably in the range from 0.08° to 0.25°, more preferably in the range from 0.1° to 0.2°.

|11| The receptacle according to any of the preceding embodiments, comprising a first aperture at a first end and a second aperture at a second end. In one aspect of this embodiment, the first aperture has a greater area than the second aperture, preferably at least 50% greater, more preferably at least 100% greater, most preferably at least 200% greater, based on the surface area of the second aperture. In an alternative embodiment, the receptacle comprises a first aperture at a first end and a dead end at a second end.

|12| The receptacle according to any of the preceding embodiments, wherein the internal diameter at $p_1$ is di and the internal diameter at $p_2$ is $d_2$ and $d_1$ is greater than $d_2$. di is preferably at least 0.05% greater than $d_2$, more preferably at least 0.1%, most preferably at least 0.2%, based on the diameter $d_2$. $d_1$ is preferably up to 5% greater than $d_2$, preferably up to 4%, more preferably up to 3%, based on the diameter $d_2$.

|13| The receptacle according to any of the preceding embodiments, comprising an attachment means at an aperture. Preferably, an attachment means is at an end, more preferably at a second end. A preferred attachment means is adapted and arranged for attaching one or both selected from the group consisting of a needle and a tube. Some preferred attachment means are a screw thread, a latch, a Luer fitting and a bayonet-style fitting.

|14| The receptacle according to any of the preceding embodiments, wherein the layer has a minimum thickness $t_{min}$ determined in the portion X of at least 60 nm, preferably at least 90 nm, more preferably at least 100 nm, most preferably at least 110 nm.

|15| A kit comprising as kit parts:

> a. the receptacle according to any of the preceding claims, and
> b. a charge;
> wherein the charge is adapted and arranged to be positioned in the interior such that:

>> i. the charge seals a cross section of the interior between the inner surfaces of the side walls;
>> ii. the charge has a front end at the axial position closest to $p_B$ at which the charge contacts the layer or the inner surface;
>> iii. the charge has a back end at the axial position closest to $p_A$ at which the charge contacts the layer or the inner surface;
>> iv. a length of the charge $L_C$ is the distance between the front end and the back end;
>> v. the charge has a charge axial position, being the axial position of the front end;

> the charge is movable in a direction parallel to the axis with a dynamic friction g, g being a function of the charge axial position.

|16| The kit according to embodiment |15|, wherein for charge axial positions in the range from $p_1 + L_C$ to $p_2$, the dynamic friction g has a maximum value $g_{max}$, a minimum value $g_{min}$ and a mean value $g_{mean}$ and the value of $g_{min}$ / $g_{max}$ is at least 0.7, preferably at least 0.8, more preferably at least 0.90, most preferably at least 0.94, most preferably at least 0.98.

|17| The kit according to embodiment |15| or |16|, wherein for charge axial positions in the range from $p_1 + L_C$ to $p_2$, the dynamic friction g has a standard deviation $g_{SD}$ less than 2 N, preferably less than 1 N, more preferably less than 0.5 N, most preferably less than 0.2 N.

|18|An assembly comprising a receptacle according to any of the embodiments |1| to |14| and a charge, wherein:

> a. The receptacle has an aperture;
> b. the charge is positioned in the interior sealing a cross section of the interior;
> c. the interior contains a liquid pharmaceutical composition, located between the sealed cross section and the aperture; and
> d. the assembly is adapted and arranged for the liquid pharmaceutical composition to be ejected through the aperture by movement of the charge in a direction parallel to the axis.

|19| A process for preparing a receptacle according to any of the claims |1| to |14|, a kit according to embodiments |15| or |16|, or an assembly according to embodiment |17|, comprising a process step of applying the layer of lubricant by spreading with a spreading tool.

|20| A use of a layer of lubricant for improving uniformity of dynamic friction in a pharmaceutical receptacle having a layer of a lubricant, wherein the ratio of the mean thickness over a first half of the layer to the mean thickness over a second half of the layer $T_A:T_B$ is in the range from 1:5 to 5:1, preferably in the range from 3:1 to 1:3, more preferably in the range from 2:1 to 1:2, most preferably in the range from 4:3 to 3:4.

Diameters, layer thicknesses and roughness

The axis of the receptacle is used to determine axial position. At a given axial position, the side wall is a perimeter having a thickness lying in a cross-sectional plane perpendicular to the axis, likewise for the lubricant layer. Internal diameter, thickness of the lubricant layer, thickness of the side wall and surface roughness at a given point along the axis are preferably mean values determined around a perimeter. A mean around a perimeter is an angular mean. An angular mean is preferably determined by measuring at 8 points around the perimeter, the 8 points being separated by equal angles.

Receptacle

A preferred receptacle is adapted and arranged to contain a pharmaceutical liquid. Some preferred receptacles are syringes, syringe barrels, cartridges, dead-end containers and vials.
A preferred receptacle has one or more apertures. An aperture is preferably located at an end of the receptacle. In one embodiment, the receptacle has one aperture. In another embodiment, the receptacle has two apertures.
The receptacle preferably has two ends, a first end and a second end.
A preferred receptacle has a first aperture at a first end. One type of preferred receptacle has a second aperture at a second end. Another type of preferred receptacle has a dead end at a second end.

Elongate Barrel Section

The receptacle has an elongate barrel section. The elongate barrel section denominates a section of the receptacle. The receptacle may have further sections outside of the elongate barrel section. Another term for an elongate barrel section is a tube section. A preferred elongate barrel section is tubular.
Possible embodiments of the elongate barrel section and the axis are described herein in mathematical terms, for example as axes of symmetry, rotation or revolution, surfaces and solids of revolution and shapes such as cylinders and truncated cones. These embodiments are to be understood as allowing some variation from these precise mathematical concepts. Suitable variations from the mathematic concepts are those which do not inhibit the receptacle from functioning as a plunger system in cooperation with a charge.
The elongate barrel section has an axis. The axis may be an axis of rotation for the elongate barrel section. The axis may be an axis of revolution for the elongate barrel section. The side wall may be a solid of revolution about the axis. The inner surface may be a surface of revolution about the axis. The layer may be a solid of revolution about the axis.
The axis defines an axial position p. The axial position p is an axial position along the axis. In this document, the symbol p denotes an axial position in general terms and specific axial positions are denoted by the letter p with a subscript.
An axial position p along the axis is used as a parameter to describe the locations of points or cross-sections along the elongate barrel section, for example on the side wall. The axial position of a point not on the axis is found by projecting the point onto the axis by a displacement vector perpendicular to the axis. A cross section is a plane perpendicular to the axis. The axial position of a cross section is found at the point where the cross section meets the axis.

The elongate barrel section extends from an axial position $p_A$ to an axial position $p_B$. The elongate barrel section is contained by a cross section at $p_A$ and a cross section at $p_B$.

The receptacle has a side wall extending over the elongate barrel section. The side wall has an inner surface. The inner surface borders an interior. Preferred shapes for the side wall are a hollow cylinder, a hollow prism and a hollow truncated cone. A preferred hollow truncated cone has a diameter which decreases from $p_A$ to $p_B$. Preferred shapes for the interior are a cylinder, a prism and a truncated cone. A preferred truncated cone has a diameter which decreases from $p_A$ to $p_B$.

The inner surface is preferably smooth, but may have some roughness.

The thickness of the side wall is preferably measured as a difference in radial distance from the axis of the inner surface and an outer surface of the side wall.

The thickness of the side wall is preferably measured as a difference in radial distance from the axis of an inner surface of the layer and the inner surface of the side wall.

Preferred materials for the side wall are polymers and glasses.

In one embodiment, the side wall comprises a polymer, preferably is made out of a polymer. The polymer is preferably one or both selected from the group consisting of: one or more cyclic olefin copolymers and one or more cyclic olefin polymers. In one aspect of this embodiment, the polymer is preferably at least 30 wt. % of the side wall, more preferably at least 50 wt. %, more preferably at least 80 wt. %, most preferably about 100 wt. %.

In one embodiment, the side wall comprises a glass, preferably is made of a glass. A preferred glass in this context comprises one or more selected from the group consisting of: silicon, boron and aluminium. One preferred glass comprises boron and silicon. One preferred glass is a borosilicate glass. One preferred glass comprises aluminium and silicon. One preferred glass is an aluminosilicate glass. In one aspect of this embodiment, the glass is preferably at least 30 wt. % of the side wall, more preferably at least 50 wt. %, more preferably at least 80 wt. %, most preferably about 100 wt. %.

Lubricant layer

The layer of lubricant is located on the inner surface of the side wall. The lubricant layer may extend over the entire elongate barrel section or just a part of it. The lubricant layer extends over the entire portion X.

A preferred lubricant is a silicone-based lubricant.

A preferred lubricant comprises one or more polysiloxanes.

A preferred lubricant comprises one or more silicone oils, preferably a total content of silicone oils in the range from 10 to 50 wt. %, more preferably in the range from 20 to 40 wt. %, most preferably in the range from 25 to 35 wt. %, based on the total weight of the lubricant. A preferred silicone oil is a poly dimethyl silicone.

A preferred lubricant comprises a crosslinked poly siloxane matrix, preferably a total content of crosslinked poly siloxane matrix in the range from 50 to 90 wt. %, more preferably in the range from 60 to 80 wt. %, most preferably in the range from 65 to 75 wt. %, based on the total weight of the lubricant.

A preferred lubricant may be prepared from a mixture comprising one or more, preferably all, of the following:

- a reactive polysiloxane
- an unreactive polysiloxane
- a catalyst
- a diluent

A preferred reactive polysiloxane is adapted and arranged to undergo a cross-linking reaction to obtain a cross-linked network. The cross-linking may be catalysed by the catalyst.

A preferred unreactive polysiloxane does not undergo a cross-linking reaction. A preferred unreactive polysiloxane comprises one or more alkyl groups. A further preferred unreactive polysiloxane is fully substituted with alkyl groups.

A preferred catalyst catalyses a reaction to cross-link polysiloxanes.

A preferred diluent solves one or more of the other constituents of the mixture. A preferred diluent is silicon based. A preferred diluent is a short chain polysiloxane, preferably having 6 repeat units or less. A preferred diluent is hexamethyl disiloxane.

A preferred lubricant contains not more than 10 wt. % water, based on the total weight of the lubricant, preferably not more than 5 wt. %, more preferably not more than 1 wt. %.

The lubricant layer has a thickness profile from pi to $p_2$.

The thickness t2 of the layer at $p_2$ is preferably similar to the thickness $t_1$ at $p_1$. Preferably, the ratio $t_2:t_1$ is in

the range from 5:1 to 1:5, preferably in the range from 3:1 to 1:3, more preferably in the range from 2:1 to 1:2, most preferably in the range from 4:3 to 3:4.

The thickness preferably has a reduced variance. According to one embodiment, the layer has a maximum layer thickness $T_{max}$ determined between $p_1$ and $p_2$, a minimum layer thickness Tmin determined between $p_1$ and $p_2$ and the ratio $T_{min} / T_{max}$ is at least 0.7, preferably at least 0.8, more preferably at least 0.9, most preferably at least 0.95, most preferably at least 0.98.

In one embodiment, the layer extends over at least 70%, more preferably at least 80%, more preferably at least 90%, most preferably about 100% of the length $L_B$ of the elongate barrel section. In another embodiment, the lubricant extends over 20 to 60 % of the length $L_B$ elongate barrel section.

In one embodiment, the lubricant layer has a minimum thickness $t_{min}$ determined between $p_1$ and $p_2$ inclusive of at least 60 nm, preferably at least 90 nm, more preferably at least 100 nm, most preferably at least 110 nm.

In one embodiment, the layer has a minimum thickness $t_{min}$ determined between $p_1$ and $p_2$ inclusive which is greater than the mean roughness of the inner surface of the side wall between $p_1$ and $p_2$, preferably at least 20 nm greater, more preferably at least 40 nm, more preferably at least 60 nm greater.

Liquid Pharmaceutical Composition

The receptacle is for pharmaceutical packaging. A preferred receptacle is adapted and arranged to contain a liquid.

A liquid pharmaceutical composition preferably comprises an active compound.

A liquid pharmaceutical composition is a fluid.

A preferred amount of liquid pharmaceutical composition is in the range from 0.1 to 100 ml, preferably in the range from 0.5 to 70 ml, more preferably in the range from 0.8 to 40 ml, most preferably in the range from 1 to 10 ml, most preferably in the range from 2 to 5 ml.

Charge

The receptacle is adapted and arranged to accommodate a charge. A preferred charge is adapted and arranged to be accommodated in the receptacle. The receptacle are preferably complementary such that the charge can be introduced into the interior of the receptacle and such that the charge can move within the interior in a direction parallel to the axis.

A preferred charge is made of an elastic material or comprises a part made of an elastic material. The charge is preferably adapted and arranged to seal a cross-section of the interior. The charge is preferably adapted and arranged to move inside the receptacle, preferably along the axis defined by the elongate extension of the receptacle. When inside the receptacle, movement of the charge is preferably resisted by a frictional force between the charge and an inside surface of the receptacle.

The charge may be attached to an elongate rod adapted and arranged to push or pull the charge in a direction parallel to the axis.

A preferred charge is a plunger.

Charge axial position

When in position in the receptacle, the charge makes contact with the layer or the inner surface or both. The front end of the charge is the point of forwardmost contact, in a direction from $p_A$ to $p_B$, of the charge with the layer or the inner surface. The back end of the charge is the point of backmost contact, in a direction from $p_A$ to $p_B$, of the charge with the layer or the inner surface. The charge axial position is the axial position of the front end.

The distance between the front end and the back end of the charge is the charge length Lc.

Kit

A contribution to at least partially solving one or more of the above-mentioned objects is made by a kit comprising a receptacle according to this disclosure and a charge according to this disclosure.

The charge and the receptacle are preferably complementary such that the charge can be accommodated in the receptacle and can move within the interior in a direction parallel to the axis.

Frictional Forces

The movement of the charge within the receptacle is accompanied by a frictional force between the charge and the inner surface of the side wall and or the layer. The frictional force comprises both a stiction which resists the setting in motion of the charge relative to the receptacle and a dynamic friction which acts whilst the charge is in movement.

The dynamic friction is dependent on the charge axial position. The dynamic friction at a given charge axial position p is preferably determined by starting the charge at charge axial position $p_1 + L_C$ and moving the charge from $p_1 + L_C$ to $p_2$ at a constant speed of 100 mm/minute. The value of dynamic friction at charge axial position p is the force required to maintain the speed of the charge at 100 mm/ minute when the charge is at charge axial position p. A method for determining the dynamic friction is presented in the figures.

Assembly

A contribution to at least partially solving at least one of the above-mentioned objects is made by an assembly comprising a receptacle, a charge and a liquid pharmaceutical composition. The charge is positioned inside the receptacle to seal a cross-section of the interior. The liquid pharmaceutical composition is present in the interior between the sealed cross-section and an aperture of the receptacle.

In one embodiment, the liquid pharmaceutical composition fills at least 50 vol. % of the interior, preferably at least 70 vol. %, more preferably 80 vol. %.

In one embodiment, the liquid pharmaceutical composition fills less than 50 vol. % of the interior, or less than 30 vol. %, or less than 20 vol. %.

The assembly is preferably adapted and arranged such that the liquid pharmaceutical composition can be ejected from the interior by moving the charge in a direction parallel to the axis towards the second end.

A preferred assembly functions as a plunger system in which the liquid pharmaceutical composition can be ejected from the receptacle through movement of the charge.

A preferred receptacle has an attachment means, preferably at an end. A preferred attachment means is adapted and arranged for attaching a needle or tube. A needle or tube may be attached to the receptacle in the assembly.

Luer Fitting

Preferred receptacles, be they receptacles as such or as part of a kit or an assembly, have a Luer fitting. A preferred Luer fitting is compatible with ISO 80369. Preferred Luer fittings are Luer lock fittings and slip tip fittings, preferably Luer lock fittings. In one embodiment, the receptacle has a Luer lock fitting. In another embodiment, the receptacle has a slip tip fitting. A preferred Luer fitting is a male Luer fitting. Preferred Luer lock fittings are one-piece Luer lock fittings and two-piece Luer lock fittings. In one embodiment, the receptacle has a one-piece Luer lock fitting. In another embodiment, the receptacle has a tow-piece Luer lock fitting.

Process for Preparation

A receptacle according to this disclosure may be prepared by providing a receptacle without a layer lubricant and applying a layer of lubricant to the inner surface of the side wall of the receptacle. Preferred methods for applying the layer are spreading and wiping, preferably with a suitable tool.

An assembly may be prepared by the following steps:

- providing a receptacle according to this disclosure;
- introducing a charge into the receptacle, preferably via a first end, preferably via a first aperture;
- introducing a liquid pharmaceutical composition into the interior of the receptacle, preferably via a second end, preferably via a second aperture.

Figures

The invention is now further elucidated by way of figures. The figures are exemplary and do not limit the scope of the invention. Some features of the figures are shown

Summary of the Figures

Figure 1 shows a cross-sectional view of a receptacle with a layer of lubricant on an inner surface of a side wall.
Figures 2a to 2g show a single-push movement of a charge in a receptacle.
Figure 3 shows a cross-sectional view through the receptacle.

Figures 4a to 4e show the process of preparing an assembly and ejecting a pharmaceutical product.
Figure 5 shows a thickness profile of the layer of lubricant.
Figure 6 shows a gliding force profile for the kit of example 1.

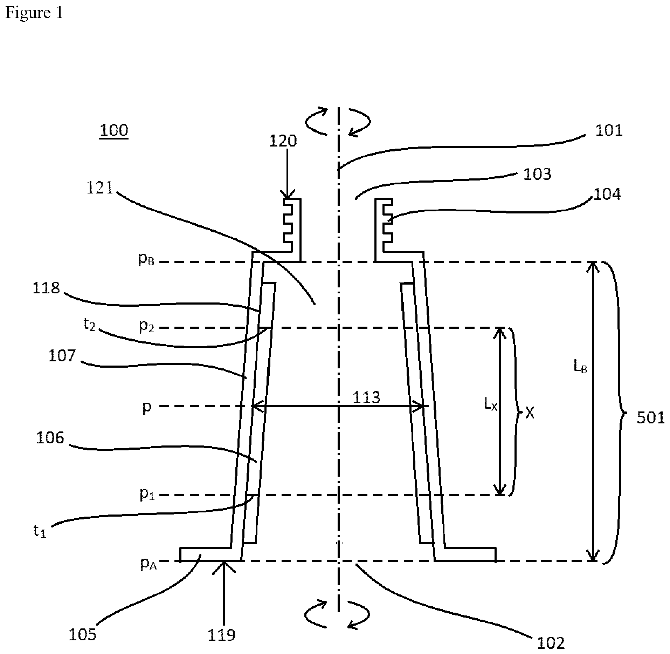

Figure 1 shows a cross-sectional view of a receptacle 100 with a lubricant layer 106 on an inner surface 118 of a side wall 107. The receptacle 100 has a first end 119 and a second end 120. At the first end 119 is a first aperture 102 and an outwardly protruding flange 105. At the second end 120 is a second aperture 103 and an attachment means 104, in this case a screw thread, for attaching a needle fitting. The receptacle 100 has an elongate barrel section 501 extending from an axial position $p_A$ to an axial position $p_B$. In this case, the elongate barrel section 501 has a hollow truncated conical shape with a greater diameter at $p_A$ than at $p_B$. The length of the elongate barrel section 501 is $L_B$. The axis 101 is in the direction of elongate extension of the receptacle 100 and is the axis of rotation of the elongate barrel section 501. The side wall 107 has an inner surface 118, on which is present a layer 106 of lubricant. The layer 106 extends over some, but not all of the side wall 107, not reaching the ends $p_A$ and $p_B$. A portion X of the elongate barrel section 501 runs between axial positions pi to $p_2$. The portion X is an abstract portion selected according to the criteria presented in the embodiments and claims. The end points $p_1$ and $p_2$ both lie within the bounds of the layer 106. The purpose of selecting the abstract portion X of the elongate barrel section 501 is to ensure that irregularities at the ends of the elongate barrel section 501 are avoided, in particular since the layer 106 may not extend all the way to the ends $p_A$ and $p_B$, as in this example. Axial positions along the receptacle 100 are measured along the axis 101. Axial positions may be given with reference to pi as a fiduciary zero point. Shown is a general axial position p as well as the internal diameter 118 between of the inner surfaces 118 of the side walls 107 at that axial position. The thicknesses $t_1$ at axial position $p_1$ and $t_2$ at axial position $p_2$ are shown.

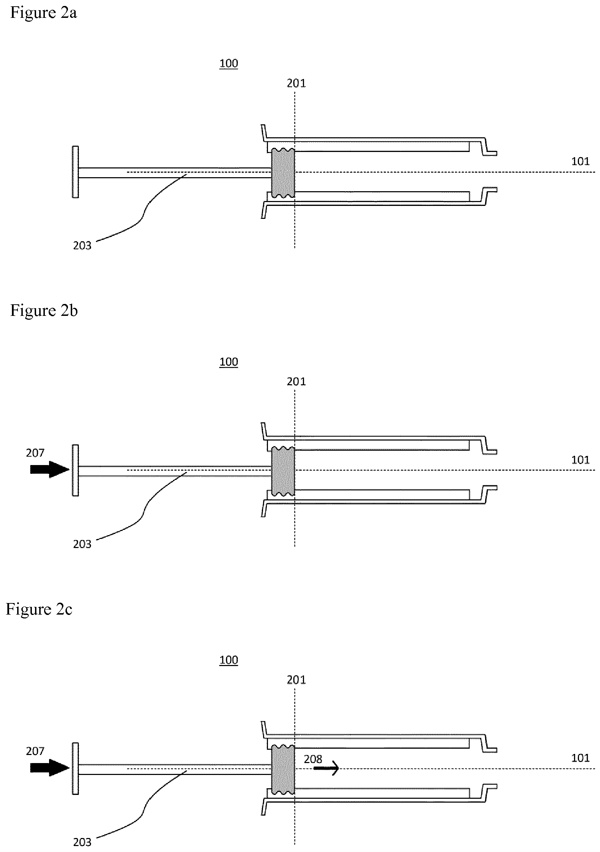

Figures 2a to 2g show a single-push movement of a charge 203 in a receptacle 100. The series of figures demonstrates how the charge 203 may be pushed in a single push along the axis 101 of the receptacle 100 at a constant speed of 100 mm/min. The process presented can also be used to construct a profile of the dynamic friction between the charge 203 and the side wall 107 / lubricant layer 106 as a function of axial position along the axis 101. Because the movement is performed in a single push, a stiction force is only relevant for the start point.

Figure 2a shows a receptacle 100 ready for single-push movement of a charge 203. The arrangement is the same as in figure 1.

Figure 2b shows the receptacle 100 of figure 2a, in which a pushing force 207 is applied to the elongate rod 202 in a direction along the axis 101. The force 207 is transferred to the charge 203. In the figure, the force 207 is inferior to the stiction force at the initial axial position 201 and the charge 203 is at rest, with the pushing force 207 cancelled out by the static frictional force between the charge 203 and the side walls 207 / lubricant layer 106. The stiction force at the axial position 201 is determined as the force 207 at which the charge 203 starts to move along the axis 101.

Figure 2c shows the receptacle 100 immediately after the pushing force 207 in figure 2b exceeds the stiction force at axial position 201 to put the charge 203 into motion. The charge 203 is depicted at the axial position 201, but in motion 208 along the axis 101. The pushing force 207 is equal to the dynamic friction at axial position 201 and the charge 203 is in a state of constant velocity along the axis 101.

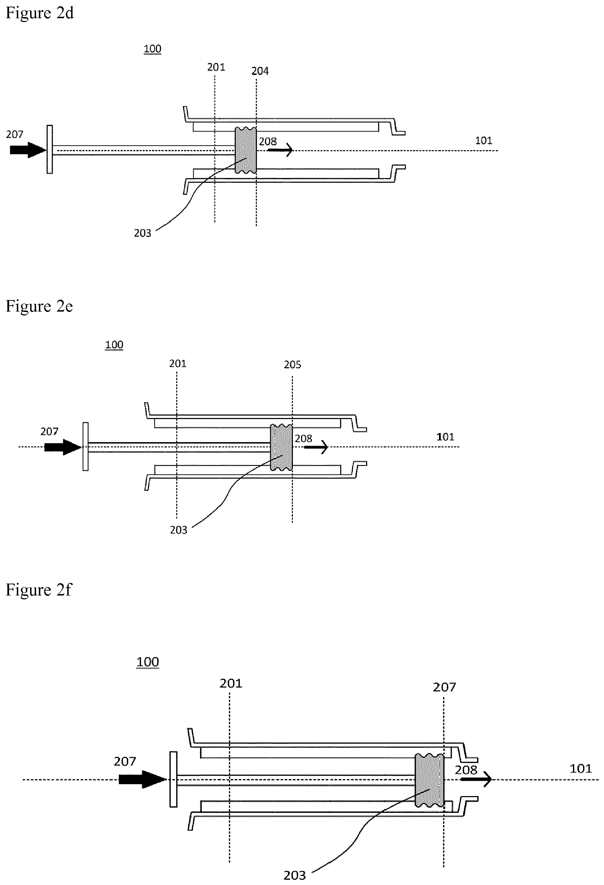

Figure 2d shows the receptacle 100 subsequent to that of figure 2c, in which the charge 203 has travelled a distance from 201 to 204 along the axis 101. The charge 203 is still in motion 208 with constant velocity with the pushing force 207 being equal to the dynamic friction at axial position 204. Therefore, the pushing force is a measure of the dynamic friction at axial position 204.

Figure 2e shows the receptacle 100 after the situation of figure 2d. The charge 203 has travelled a further distance from 204 to 205 along the axis 101. The charge is still in motion with constant velocity with the pushing force 207 and the dynamic friction being equal. The pushing force 207 is thus a measure of the dynamic friction at axial position 205.

Figure 2f shows the receptacle 100 after the situation of figure 2e. The charge 203 has travelled a further distance from 205 to 206 along the axis 101. The charge is still in motion with constant velocity with the pushing force 207 and the dynamic friction being equal. The pushing force 207 is thus a measure of the dynamic friction at axial position 206.

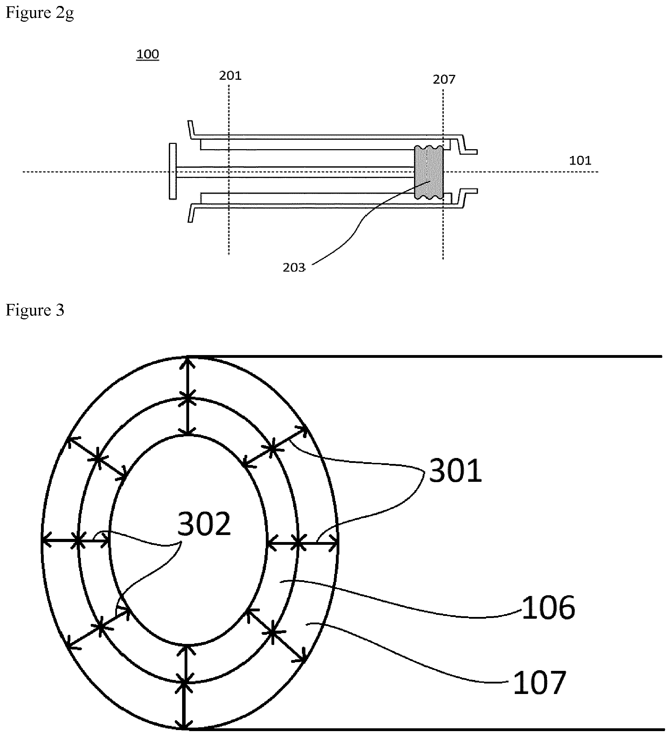

Figure 2g shows the receptacle in which the pushing force has been released at axial position 206. The axial position 206, is where the front end of the charge has arrived at $p_2$, close to the end of the lubricated layer and is in its final rest axial position.

The dynamic friction at any point along the axis 101 is provided directly as the pushing force 207 required at that point to maintain constant velocity (100 mm/min) of the charge 203 along the axis 101. The dynamic friction at points 201, 204, 205 and 206 are measured at the stages of figures 2c, 2d, 2e and 2f respectively.

Figure 3 shows a cross-sectional view through the receptacle 100 at an axial position p along the axis 101. The side wall 107 and the lubricant layer 106 are shown as concentric circular bands. The thickness 301 of the side wall 107 and the thickness 302 of the lubricant layer 106 are each shown at 8 equidistant points around the circle. A thickness of a side wall 107 or a layer 106 of lubricant at an axial position p is a mean of the thickness around the circle. This is measured as the mean of a number of equally spaced sample points around the circle, in this case 8.

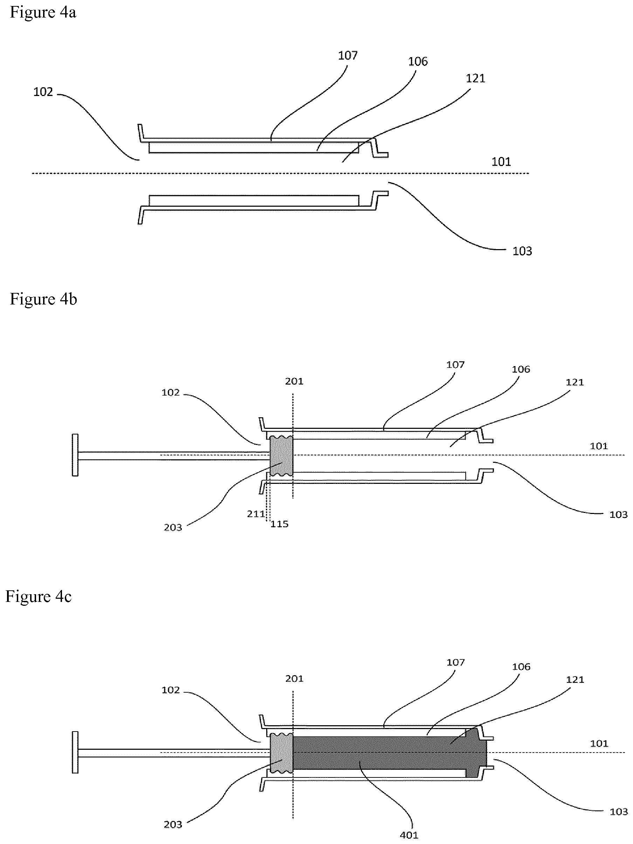

Figures 4a to 4e show the process of preparing an assembly and ejecting a pharmaceutical product 401.

Figure 4a shows a receptacle 100, ready for forming an assembly. The receptacle 100 has an axis of rotation 101, a side wall 107 and an interior 121. A layer 106 of lubricant is present on the inside of the receptacle 100. The receptacle 100 has a first aperture 102 and a second aperture 103.

Figure 4b shows the receptacle 100 of figure 4a with a charge 203 located in the interior 121. The front end of the charge 203 is at an axial position 201 along the axis, close to the first aperture 102. The back end of the charge 203 is at $p_1$, close to the start of the lubricant layer 107.

Figure 4c shows the receptacle 100 of figure 4b after having been filled with a liquid pharmaceutical composition 401. The liquid pharmaceutical composition 401 is located in the interior 121 between the front end of the charge 201 and the second aperture 103. In this form, the receptacle 100, the charge 203 and the liquid pharmaceutical composition 401 constitute and assembly.

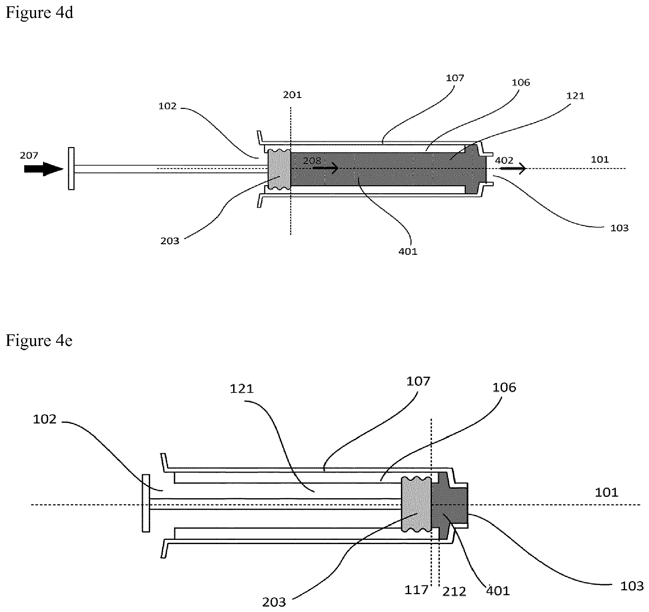

Figure 4d shows the assembly of figure 4c in which a pushing force 207 is applied to push the charge 203 along the axis 101 in a direction from the first aperture 102 towards the second aperture 103. The motion 208 of the charge 203 forces the liquid pharmaceutical composition 401 to be ejected 402 from the receptacle 100.

Figure 4e shows the assembly of figure 4d once the charge 203 has travelled along the axis 101 in a direction from the first aperture 102 towards the second aperture 103 to arrive at axial position $p_2$ near the end of the layer 106 of lubricant. The liquid pharmaceutical composition 401 has been ejected via the second aperture 103 and only a small quantity remains in the tip of the receptacle at the second aperture 103.

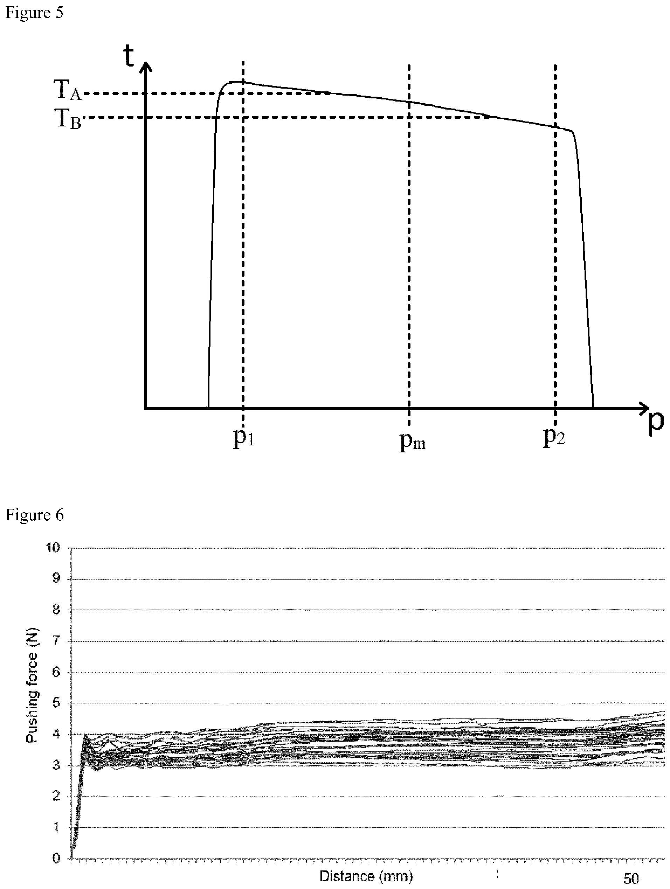

Figure 5 shows a thickness profile of the lubricant layer 106 against position partitioned along the position axis. The thickness of the layer 106 drops off rapidly at the endpoints. The portion X from $p_1$ to $p_2$ is thus displaced slightly from the end positions of the layer (106) in order to avoid these end effects. The thickness is well behaved over the portion X. $T_A$ is the mean thickness for the section extending from $p_1$ to the midpoint $p_m$. $T_B$ is the mean thickness for the section extending from $p_m$ to $p_2$. Even were the thickness profile to exhibit local variation over short ranges, the long-range slope of the distribution would be apparent from the means $T_A$ and $T_B$. According to the invention, a relatively flat thickness profile is preferred, as expressed by the relevant features recited in the claims and embodiments. In this case, it can be seen that the thickness profile decreases lightly in a direction from $p_1$ to $p_2$. Possible alternatives are a flat thickness profile without a gradient or a thickness profile which increases lightly in a direction from pi to $p_2$. A thickness profile having a large gradient, either increasing or decreasing from $p_1$ to $p_2$ is not preferred according to the invention.

Figure 6 shows a dynamic friction profile for the kit of example 1. The dynamic friction profile is determined using the procedure as presented in the figures 2a to 2g. The test was performed multiple times with fresh kits (all lines shown in the figure). In each run, a smooth level dynamic friction profile was observed with a value in the range from around 3 to 4 N, varying slightly between individual runs. The smooth level dynamic friction profile is well suited to be employed in a single-dose syringe and is particularly favourable for use in automated delivery systems.

Test Methods

Layer thickness

The thickness of the layer is determined by optical interference measurements using the RapID Explorer available from rap.ID Particle Systems GmbH. Measurements are taken from outside the receptacle through the side wall. The device is operated with the proprietary software and in accordance with the 2014 proprietary instruction manual.

Surface Roughness

Surface roughness of the inner surface of the side wall is measured using a white-light interference microscope. An area of the sample of $2\mu m$ by $2\mu m$ is scanned in tapping mode, scanning the area with 256 lines per picture and 256 dots per line. The scan rate is 0.7 Hz. The cantilever has a tip with a tip radius of $\leq 10nm$. The sample's topography is measured by evaluating the change of the amplitude of the oscillating cantilever when scanning the surface. The raw data is levelled by a line fit, using a $3^{rd}$ order polynomial fit. The root mean squared roughness $R_{rms}$ is calculated by the

$$R_{rms} = \sqrt{\frac{1}{n}\sum_{i=1}^{n} y_i^2},$$

AFM's software using the formula where n = 256 * 256 = 65536 and $y_i$ is the height value at each of the 65536 measured positions.

Examples

The following examples are for further elucidation of the invention and do not limit the scope of the claimed invention.

A receptacle was provided according to figure 1. The receptacles was a 1 ml lg TopPac available from Schott AG Germany. The inner surface of the side walls of the barrel section was coated with a silicone coating, extending up to 1 mm from each of the two ends of the barrel section. The thickness profile of the applied layer was according to table 1. The receptacle was tested by introducing a charge made of elastomer with an attached elongate rod according to figure 2a, charge FM 257/2 available from Dedecke GmbH, Germany. A process similar to that displayed in figures 2a to 2g, with measurement at 5, 15, 30 and 45 mm along the barrel, was performed to determine the dynamic friction along the barrel. The constant velocity of the charge during the measurement movement was 100 mm/min.

The root mean square of the roughness of the inner surface of the side wall over the section from $p_1$ to $p_2$ was determined to be 62 nm.

Table 1

| Example | Distance along barrel [mm] | | | | | | |
|---|---|---|---|---|---|---|---|
| | 0 | 10 | 20 | 30 | 40 | 50 | 60 |
| | Thickness t [nm] | | | | | | |
| 1 | 704 | 699 | 680 | 713 | 728 | 684 | 705 |
| 2 | 2011 | 2001 | 1988 | 2004 | 1992 | 2014 | 2033 |
| 3 | 708 | 679 | 663 | 665 | 654 | 647 | 641 |
| 4 | 69 | 287 | 714 | 1008 | 1274 | 1678 | 1914 |
| 5 | 2113 | 1711 | 1188 | 1012 | 698 | 301 | 74 |

Table 2

| Example | Distance along barrel [mm] | | | |
|---|---|---|---|---|
| | 5 | 15 | 30 | 45 |
| | Dynamic friction [N] | | | |
| 1 | 4.1 | 4.3 | 4.1 | 4.2 |
| 2 | 3.8 | 3.8 | 3.9 | 3.7 |
| 3 | 4.0 | 4.2 | 4.2 | 4.1 |
| 4 | 6.1 | 4.8 | 4.4 | 3.8 |
| 5 | 5.6 | 6.4 | 6.3 | 7.0 |

Reference List

| 100 | Receptacle |
| 101 | Axis |
| 102 | First aperture |
| 103 | Second aperture |
| 104 | Attachment means at front end of receptacle |
| 105 | Outwardly protruding flange of receptacle |
| 106 | Layer of lubricant |
| 107 | Side wall of receptacle |
| 118 | Inner surface of side wall |
| 119 | First end of receptacle |
| 120 | Second end of receptacle |

| 121 | Interior of receptacle |
| 201 | Initial charge axial position |
| 202 | Elongate rod for pushing charge |
| 203 | Charge |
| 204 | First intermediate charge axial position |
| 205 | Second intermediate charge axial position |
| 206 | Final charge axial position |
| 207 | Pushing force |
| 208 | Movement of charge along axis |
| 210 | Thickness of layer at a general axial position |
| 301 | Thicknesses of the side wall |
| 302 | Thicknesses of the layer |
| 401 | Liquid pharmaceutical composition |
| 402 | Ejection of liquid pharmaceutical composition |
| 501 | Elongate barrel section |

**Claims**

1. A receptacle (100) for pharmaceutical packaging, the receptacle (100) having an elongate barrel section (501), wherein:

> a. the elongate barrel section (501) has a direction of elongate extension and an axis (101) in the direction of elongate extension;
> b. an axial position p is determined along the axis (101);
> c. the elongate barrel section (501) extends from an axial position $p_A$ to an axial position $p_B$;
> d. an elongate barrel section length $L_B$ is the distance between $p_A$ and $p_B$;
> e. the receptacle (100) has a side wall (107) extending over the elongate barrel section (501), the side wall (107) having an inner surface (118) bordering an interior (121), the interior (121) having a diameter (113);
> f. a layer of a lubricant (106) is located on at least a part of the inner surface (118);
> g. at a given axial position p on the axis (101) between $p_A$ and $p_B$, the following:
>
>> i. the thickness of the side wall (107),
>> ii. the thickness of the layer (106), and
>> iii. the diameter of the interior (113),
>
> are each determined as an angular mean in a cross-sectional plane perpendicular to the axis (101) at the axial position p;
> h. a portion X of the axis (101) extends from an axial position $p_1$ to an axial position $p_2$ such that the following criteria are satisfied:
>
>> i. both $p_1$ and $p_2$ lie between $p_A$ and $p_B$,
>> ii. a portion length $L_X$ is the distance between $p_1$ and $p_2$,
>> iii. $L_X$ is at least a quarter of $L_B$, and
>> iv. the layer (106) extends over the entire portion X;
>
> i. $T_{mean}$ is a mean thickness of the layer (106) determined in the portion X;
> j. a position $p_m$ is the midpoint between $p_1$ and $p_2$;
> k. $T_A$ is a mean thickness of the layer (106) for a section from pi to $p_m$;
> l. $T_B$ is a mean thickness of the layer (106) for a section from $p_m$ to $p_2$;
> m. The ratio $T_A:T_B$ is in the range from 5:1 to 1:5;
> n. one or more criteria selected from the group consisting of:
>
>> i. The length $L_B$ is in the range from 3 to 20 cm;
>> ii. The mean value of the diameter (113) of the interior (121) determined over the range $p_A$ to $p_B$ is in the range from 0.4 to 4 cm;
>> iii. The mean thickness of the sidewall (107) determined over the range $p_A$ to $p_B$ is in the range from 0.3 to 4 mm;

iv. The volume of the interior (121) is in the range from 0.1 to 150 ml are satisfied.

2. The receptacle (100) according to claim 1, wherein the mean thickness $T_{mean}$ of the layer (106) is in the range from 100 nm to 3000 nm.

3. The receptacle according to claim 1 or 2, wherein:

   a. the inner surface of the side wall has a root mean squared surface roughness $r_{rms}$, and
   b. the ratio $r_{rms} : T_{mean}$ is in the range from 1:2 to 1:20.

4. The receptacle according to any of the preceding claims, wherein the layer has a maximum layer thickness $T_{max}$ determined between pi and $p_2$, a minimum layer thickness Tmin determined between $p_1$ and $p_2$ and the value of $T_{min} / T_{max}$ is at least 0.7.

5. The receptacle (100) according to any of the preceding claims, wherein the side wall (107) comprises a plastic or a glass or both.

6. The receptacle (100) according to any of the preceding claims, wherein the lubricant comprises one or more silicone oils.

7. The receptacle (100) according to claim 6, wherein the one or more silicone oils are at least partially contained in a matrix.

8. The receptacle (100) according to any of the preceding claims, wherein the interior (121) is cylindrical or truncated conical over the elongate barrel section (501).

9. The receptacle (100) according to any of the preceding claims, comprising a first aperture (102) at a first end (119) and a second aperture (103) at a second end (120).

10. The receptacle (100) according to any of the preceding claims, comprising an attachment means (104) at an aperture.

11. A kit comprising as kit parts:

   c. the receptacle (100) according to any of the preceding claims, and
   d. a charge (203);
   wherein the charge (203) is adapted and arranged to be positioned in the interior (121) such that:

      i. the charge seals a cross section of the interior (121) between the inner surfaces (118) of the side walls (107);
      ii. the charge (203) has a front end at the axial position closest to $p_B$ at which the charge contacts the layer (106) or the inner surface (118);
      iii. the charge (203) has a back end at the axial position closest to $p_A$ at which the charge (203) contacts the layer (106) or the inner surface (118);
      iv. a length of the charge Lc is the distance between the front end and the back end;
      v. the charge (203) has a charge axial position, being the axial position of the front end;

   the charge is movable in a direction parallel to the axis (101) with a dynamic friction g, g being a function of the charge axial position.

12. The kit according to claim 11, wherein for charge axial positions in the range from pi + $L_C$ to $p_2$, the dynamic friction g has a standard deviation $g_{SD}$ less than 2 N.

13. An assembly comprising a receptacle (100) according to any of the claims 1 to 10 and a charge (203), wherein:

   e. The receptacle (100) has an aperture (103);
   f. the charge (203) is positioned in the interior (121) sealing a cross section of the interior (121);
   g. the interior (121) contains a liquid pharmaceutical composition, located between the sealed cross section and the aperture (103); and
   h. the assembly is adapted and arranged for the liquid pharmaceutical composition to be ejected through the aperture (103) by movement of the charge (203) in a direction parallel to the axis (101).

14. A process for preparing a receptacle according to any of the claims 1 to 10, a kit according to claim 11 or 12, or an assembly according to claim 13, comprising a process step of applying the layer (106) of lubricant by spreading with a spreading tool.

15. A use of a layer of lubricant (106) for improving uniformity of dynamic friction in a pharmaceutical receptacle (100) having a layer (106) of a lubricant, wherein the ratio of the mean thickness over a first half of the layer (106) to the mean thickness over a second half of the layer (106) $T_A:T_B$ is in the range from 1:5 to 5:1.

Figure 1

Figure 2a

100

201

203

101

Figure 2b

100

201

207

203

101

Figure 2c

100

201

207

208

203

101

Figure 2d

Figure 2e

Figure 2f

Figure 2g

100

201

207

101

203

Figure 3

301

302

106

107

Figure 4a

Figure 4b

Figure 4c

Figure 4d

Figure 4e

Figure 5

Figure 6

Europäisches
Patentamt

European
Patent Office

Office européen
des brevets

## EUROPEAN SEARCH REPORT

Application Number

EP 19 21 0764

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | WO 2015/181173 A1 (BECTON DICKINSON FRANCE [FR]) 3 December 2015 (2015-12-03)<br>* page 5, line 20 - page 13, line 9; figure 3 * | 1-11, 13-15<br>12 | INV.<br>A61M5/31 |
| Y | EP 2 216 061 A1 (DAIKYO SEIKO LTD [JP]) 11 August 2010 (2010-08-11)<br>* paragraph [0017] - paragraph [0043]; figures 2,6 * | 12 | |

-----

-----

**TECHNICAL FIELDS
SEARCHED (IPC)**

A61M

The present search report has been drawn up for all claims

| Place of search | Date of completion of the search | Examiner |
|---|---|---|
| Munich | 31 January 2020 | Feber, Laurent |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    .........................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 21 0764

This annex lists the patent family members relating to the patent documents cited in the above-mentioned European search report.
The members are as contained in the European Patent Office EDP file on
The European Patent Office is in no way liable for these particulars which are merely given for the purpose of information.

31-01-2020

| Patent document cited in search report | | | Publication date | Patent family member(s) | | Publication date |
|---|---|---|---|---|---|---|
| WO 2015181173 | | A1 | 03-12-2015 | CA | 2949881 A1 | 03-12-2015 |
| | | | | CN | 106413782 A | 15-02-2017 |
| | | | | EP | 3148620 A1 | 05-04-2017 |
| | | | | JP | 2017519553 A | 20-07-2017 |
| | | | | KR | 20170008296 A | 23-01-2017 |
| | | | | US | 2017182252 A1 | 29-06-2017 |
| | | | | WO | 2015181173 A1 | 03-12-2015 |
| EP 2216061 | | A1 | 11-08-2010 | EP | 2216061 A1 | 11-08-2010 |
| | | | | JP | 5344252 B2 | 20-11-2013 |
| | | | | JP | WO2009057378 A1 | 10-03-2011 |
| | | | | US | 2010305513 A1 | 02-12-2010 |
| | | | | WO | 2009057378 A1 | 07-05-2009 |

EPO FORM P0459

For more details about this annex : see Official Journal of the European Patent Office, No. 12/82

**REFERENCES CITED IN THE DESCRIPTION**

**Patent documents cited in the description**

- US 4767414 A

- EP 0920879 B1